# EUROPEAN PATENT SPECIFICATION

(11) **EP 4 023 145 B1**
(45) Date of publication and mention of the grant of the patent: **21.02.2024**
(21) Application number: 21189629.5
(22) Date of filing: 04.08.2021
(51) Int. Cl.: A61B 5/00, A61B 5/145, A61B 5/1455

(54) **APPARATUS AND METHOD FOR ESTIMATING BIO-INFORMATION**
VORRICHTUNG UND VERFAHREN ZUR SCHÄTZUNG VON BIOINFORMATIONEN
APPAREIL ET PROCÉDÉ D'ESTIMATION D'INFORMATIONS BIOLOGIQUES

(30) Priority: 05.01.2021 KR 20210010078
(43) Date of publication of application: 06.07.2022
(73) Proprietor: Samsung Electronics Co., Ltd., Suwon-si, Gyeonggi-do 16677 (KR)
(72) Inventor: Park, Jin Young, Hwaseong-si (KR); Jung, Myoung Hoon, Bucheon-si (KR); Kim, Sang Kyu, Yongin-si (KR); Kim, Yoon Jae, Seoul (KR); Moon, Hyun Seok, Hwaseong-si (KR); Ahn, Sung Mo, Yongin-si (KR); Eom, Kun Sun, Yongin-si (KR)
(74) Representative: Grünecker Patent- und Rechtsanwälte PartG mbB

(56) References cited:
- US-A1- 2017 337 413
- US-A1- 2019 200 866
- US-A1- 2019 380 586

## Description

### BACKGROUND

### 1. Field

The disclosure relates to an apparatus and a method for estimating bio-information, and more particularly to technology for non-invasively estimating antioxidant levels.

### 2. Description of Related Art

Reactive oxygen species act as a biological defense factor such as white blood cells protecting the body against infections. However, it has been known that excessive generation of reactive oxygen species in the body may lead to various tissue diseases. Common factors that cause the reactive oxygen species include stress, alcohol, peroxides, medicine, and the like. The reactive oxygen species produced by these factors may cause cranial nerve diseases, circulatory diseases, cancer, digestive tract diseases, liver diseases, arteriosclerosis, renal diseases, diabetes, aging, and the like.

Our bodies have a series of antioxidant defense systems to protect against oxygen toxicity. For normal operation of the systems, sufficient antioxidants such as vitamin E, vitamin C, carotenoid, flavonoid, and the like may be consumed, and as many foods that are rich in antioxidants as possible may be eaten for an effective antioxidant action. Accordingly, there may be a need for an apparatus for easily identifying the amount of antioxidants in the body.
US 2017/337413 A1 discloses a bio-sensor device, integrated with a display portion, which includes a surface for touching by a body part, such as a finger. A light source, such as an array of LEDs, emit light through the surface so as to be reflected and partially absorbed by the body part An array of photodetectors detects light reflected back by the body part and generates signals corresponding to an image of the light reflection, which corresponds to the light absorption pattern in the body part. The light absorption pattern may correlate to a fingerprint, a blood vessel pattern, blood movement within the blood vessels, combinations thereof, or other biometric feature. A processor receives the signals from the photodetectors and analyzes the signals to determine a characteristic of the body part. The characteristic may be used to authenticate the user of the bio-sensor device by comparing the detected characteristic to a stored characteristic.
US 2019/380586 A1 discloses an electronic device which includes a housing, light emitting diodes (LEDs) of a first number, photodetectors of a second number, and a control circuit operatively connected to the LEDs and the photodetectors. The control circuit is configured to cause the LEDs of a third number to emit light sequentially or simultaneously and to cause the photodetectors of a fourth number to detect light from the LEDs of the third number. The third number is 1 or 2. The fourth number is one of an integer that is not less than 2 and is not greater than the second number.

### SUMMARY

It is therefore the object of the present invention to provide an improved apparatus for estimating bio-information, a corresponding method and a corresponding computer-readable storage medium storing instructions that an apparatus to perform a corresponding method. This object is solved by the subject matter of the independent claims.

Preferred embodiments are defined by the dependent claims.

In accordance with an aspect of the disclosure, there is provided an apparatus for estimating bio-information, the apparatus including a display including unit pixels, each of the unit pixels including light sources configured to emit light having different wavelengths, and one or more detectors configured to detect light having the different wavelengths. The apparatus further includes a processor configured to detect a contact position of an object on the display, determine a partial area of the display, based on the detected contact position, determine source pixels configured to emit light onto an object, among the unit pixels, based on the determined partial area, determine detector pixels configured to detect light that is scattered or reflected from the object, among the unit pixels, based on the determined partial area control the determined source pixels and the determined detector pixels to obtain a spectrum based on the light having multiple wavelengths that is detected by the detector pixels, and estimate bio-information, based on the obtained spectrum.

The light sources may include a red light source, a green light source, and a blue light source.

The detectors may include either one or both of a photodiode and a Complementary Metal Oxide Semiconductor (CMOS) image sensor.

The light sources and the detectors may be disposed on a same surface of a pixel circuit board.

The light sources and the detectors may be patterned on the same surface of the pixel circuit board.

A partition wall for blocking light may be interposed between the light sources and the detectors.

The light sources may be disposed on a first surface of the pixel circuit board, and the detectors may be disposed on a second surface of the pixel circuit board, the second surface being opposite to the first surface.

The processor may be further configured to determine the source pixels and the detector pixels at different positions, among the unit pixels.

The processor may be further configured to determine the source pixels and the detector pixels, based on any one or any combination of a contact position of the object, a type of the bio-information, a light path, and an allowable range of Signal-to-Noise Ratio (SNR) values.

The processor may be further configured to perform color modulation of the light sources of the determined source pixels, based on a type of the bio-information.

The processor may be further configured to perform the color modulation of the light sources of the determined source pixels, to include a wavelength range of 470 nm to 510 nm.

The processor may be further configured to extract a light intensity for each of the different wavelengths, based on Full Width Half Maximum (FWHM) characteristics of the light sources emitting light of each wavelength, and obtain the spectrum, based on the extracted light intensity for each of the different wavelengths.

The bio-information may include any one or any combination of skin carotenoid, blood carotenoid, glucose, urea, lactate, triglyceride, total protein, cholesterol, and ethanol.

In accordance with an aspect of the disclosure, there is provided a method of estimating bio-information, the method including, detecting a contact position of an object on a display including unit pixels, each of the unit pixels including light sources emitting light having different wavelengths and one or more detectors detecting light having the different wavelengths, determining a partial area of the display, based on the detected contact position, determining source pixels and detector pixels, among the unit pixels, based on the determined partial area, and controlling the determined source pixels to emit light of onto the object. The method further includes controlling the determined detector pixels to detect light that is scattered or reflected from the object, and obtaining a spectrum based on the light having multiple wavelengths that is detected by the detector pixels, and estimating bio-information, based on the obtained spectrum.

The light sources may include a red light source, a green light source, and a blue light source.

The light sources and the detectors may be disposed on a same surface of a pixel circuit board.

The light sources and the detectors may be patterned on the same surface of the pixel circuit board.

A partition wall for blocking light may be interposed between the light sources and the detectors.

The light sources may be disposed on a first surface of the pixel circuit board, and wherein the detectors are disposed on a second surface of the pixel circuit board, the second surface being opposite to the first surface.

The determining of the source pixels and the detector pixels may include determining the source pixels and the detector pixels at different positions, among the unit pixels.

The determining of the source pixels and the detector pixels may include determining the source pixels and the detector pixels, based on any one or any combination of a contact position of the object, a type of the bio-information, a light path, and an allowable range of Signal-to-Noise Ratio (SNR) values.

The controlling of the determined source pixels may include performing color modulation of the light sources of the determined source pixels, based on a type of the bio-information.

The controlling of the determined source pixels may include performing the color modulation of the light sources of the determined source pixels, to include a wavelength range of 470 nm to 510 nm.

The obtaining of the spectrum may include extracting a light intensity for each of the different wavelengths, based on Full Width Half Maximum (FWHM) characteristics of the light sources emitting light of each wavelength, and obtaining the spectrum, based on the extracted light intensity for each of the different wavelengths.

In accordance with an aspect of the disclosure, there is provided an apparatus for estimating bio-information, the apparatus including a display including unit pixels, each of the unit pixels including light sources configured to emit light having different wavelengths, and at least one detector configured to detect light having the different wavelengths. The apparatus further includes a processor configured to detect a contact position of an object on the display, determine a partial area of the display, based on the detected contact position, determine source pixels configured to emit light onto the object, among the unit pixels, based on the determined partial area, determine detector pixels configured to detect light that is scattered or reflected from the object, among the unit pixels, based on the determined partial area, control the determined source pixels and the determined detector pixels to obtain a spectrum based on the light having multiple wavelengths that is detected by the detector pixels, and estimate bio-information, based on the obtained spectrum.

### BRIEF DESCRIPTION OF THE DRAWINGS

The above and other aspects, features, and advantages of embodiments of the disclosure will be more apparent from the following description taken in conjunction with the accompanying drawings.
FIG. 1 is a block diagram of an apparatus for estimating bio-information, according to an embodiment.
FIGS. 2A, 2B and 2C are diagrams explaining examples of a display, according to embodiments.
FIGS. 3A and 3B are diagrams explaining examples of driving unit pixels and generating spectra, according to embodiments.
FIG. 4 is a diagram explaining another example of a display, according to an embodiment.
FIG. 5 is a block diagram of an apparatus for estimating bio-information, according to another embodiment.
FIG. 6 is a flowchart of a method of estimating bio-information, according to an embodiment.
FIGS. 7 and 8 are diagrams illustrating examples of wearable devices having an apparatus for estimating bio-information according to embodiments.

### DETAILED DESCRIPTION

Details of embodiments are included in the following detailed description and drawings. Advantages and features of example embodiments, and a method of achieving the same will be more clearly understood from the following embodiments described in detail with reference to the accompanying drawings. Throughout the drawings and the detailed description, unless otherwise described, the same drawing reference numerals will be understood to refer to the same elements, features, and structures.

It will be understood that, although the terms first, second, etc. may be used herein to describe various elements, these elements may not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'unit' or 'module', etc., may be understood as a unit that performs at least one function or operation and that may be embodied as hardware, software, or a combination thereof.

Hereinafter, embodiments of an apparatus and a method for estimating bio-information will be described in detail with reference to the accompanying drawings. The embodiments of the apparatus for estimating bio-information that will be described below may be mounted in a smartphone, a tablet PC, a wearable device, a desktop computer, a laptop computer, and medical equipment in medical institutions and the like.

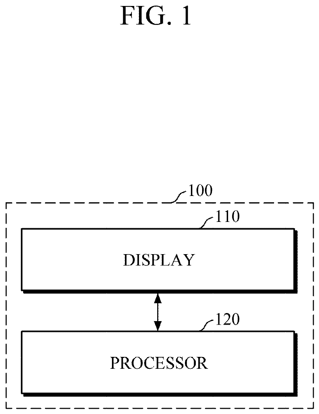

FIG. 1 is a block diagram of an apparatus for estimating bio-information, according to an embodiment.

Referring to FIG. 1, the apparatus 100 for estimating bio-information includes a display 110 and a processor 120.

The display 110 may include a plurality of unit pixels capable of displaying images and/or measuring light signals. In this case, the unit pixel may be an Organic Light Emitting Diodes (OLED)-based pixel that emits light by itself without having a separate backlight. For example, each unit pixel may include light sources emitting red, green, and blue light, and may display images with one or a combination of the colors, or may emit light onto an object to measure an optical signal.

In addition, each unit pixel may include one or more detectors for detecting light. The detector may detect light and may convert the detected light signal into an electric signal and output the signal. The detector may be a photodiode, but is not limited thereto and may be a photo transistor (PTr) or an image sensor (e.g., Complementary Metal Oxide Semiconductor (CMOS) image sensor).

In addition, the display 110 may include a window (e.g., glass substrate) for passing external light or light input by the light sources of the unit pixels. In this case, the window may be formed as a smooth flat surface, a curved surface, etc., so that an object (e.g., finger) may come into contact therewith.

The processor 120 may control the light source and the detector of the unit pixel when the object comes into contact with the window of the display 110. For example, the processor 120 may determine one or more source pixels and one or more detector pixels among the plurality of unit pixels, and may measure light signals from the object by controlling light sources of the determined source pixels and detectors of the detector pixels. By considering a contact position of the object, a type of bio-information to be estimated, a light path defined for estimating bio-information, an allowable range of Signal-to-Noise Ratio (SNR) values, etc., the processor 120 may determine the source pixels and the detector pixels, in which case the processor 120 may determine the source pixels and the detector pixels at different positions.

By modulation of various colors, the processor 120 may drive the light sources of the source pixels to emit light of a predetermined wavelength. In this case, a color modulation pattern may be pre-defined by considering a type of bio-information, a measurement position of the object, or the like. In this case, the color modulation pattern may be defined to include a wavelength range according to the type of bio-information. For example, in the case in which an antioxidant index is detected, the color modulation pattern may be defined to include at least a wavelength range of 470 nm to 510 nm.

The processor 120 may sequentially or simultaneously drive multi-wavelength light sources of the source pixels. For example, the processor 120 may drive the light sources of the source pixels in an order from short to long wavelengths or vice versa, or in a predefined driving order such as a random order and the like. In this case, upon selecting a plurality of source pixels, the processor 120 may drive the plurality of source pixels simultaneously, sequentially, or in a predetermined pattern. Information, such as a driving order of the source pixels, power intensity and duration of the light sources, and the like may be pre-defined.

The processor 120 may receive signals output from the detectors of the detector pixels, and may estimate bio-information based on the received signals. The processor 120 may generate a spectrum based on the intensity of light of each wavelength that is detected by the detector of the detector pixel. For example, if light of multiple wavelengths is detected by the plurality of detector pixels, the processor 120 may generate spectra for the respective detector pixels. In this case, the processor 120 may extract a light intensity for each wavelength based on Full Width Half Maximum (FWHM) characteristics of the light source that emits light in each wavelength range that is detected by the detector pixels, and may generate spectra for all of the wavelengths based on the extracted light intensity for each wavelength. However, example embodiments are not limited thereto.

In addition, the processor 120 may extract features from the generated spectra, and may estimate bio-information by using the extracted features and a pre-defined estimation model. In this case, bio-information may include, for example, skin carotenoid and blood carotenoid that are associated with antioxidant levels. However, the bio-information is not limited thereto, and may include glucose, urea, lactate, triglyceride, total protein, cholesterol, and ethanol.

Further, the processor 120 may perform user authentication by using the signals detected by the detector pixels. For example, the processor 120 may detect a user's fingerprint information by analyzing the detected signals, and may perform user authentication by using the detected fingerprint information. In addition, the processor 120 may proceed to estimating bio-information for a user having been authenticated.

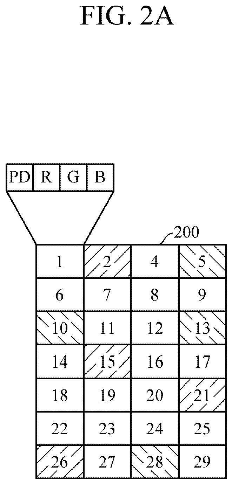

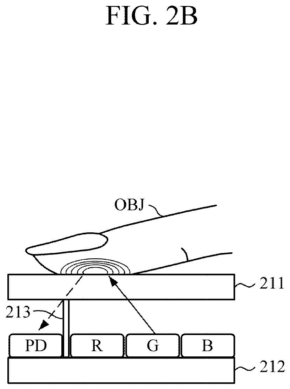

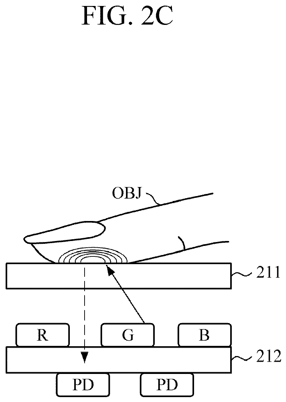

FIGS. 2A, 2B and 2C are diagrams explaining examples of a display, according to embodiments.

FIG. 2A is a diagram illustrating an example of an arrangement of unit pixels of a display. Referring to FIG. 2A, a plurality of unit pixels of the display 110 may be arranged in a rectangular array 200. However, the arrangement is not limited thereto, and may be a circular, rectangular, and pentagonal shape or the like and may vary according to a size, shape, and the like of the display. As illustrated in FIG. 2A, each unit pixel may include red R, green G, and blue B light sources and the detector PD. While FIG. 2A illustrates 29 unit pixels, but the number is not limited thereto.

FIGS. 2B and 2C are diagrams explaining an arrangement structure of light sources and detectors of each unit pixel of the display 110.

According to an embodiment, as illustrated in FIG. 2B, each unit pixel of the display 110 may have multi-wavelength light sources R, G, and B and the detector PD that are disposed on the same surface. For example, the multi-wavelength light sources R, G, and B and the detector PD may be disposed on a top surface of a pixel circuit board 212. In this case, the multi-wavelength light sources R, G, and B and the detector PD may be patterned on the same surface of the pixel circuit board 212. In this case, the multi-wavelength light sources R, G, and B may be disposed in the order of red R, green G, and blue B, starting from the detector PD, but the order of arrangement is not particularly limited thereto.

As illustrated herein, when an object OBJ comes into contact with a window 211 of a display 110, the multi-wavelength light sources R, G, and B may be driven with multicolor modulation under the control of the processor 120, to emit light of multiple wavelengths onto the object OBJ, and light scattered or reflected from the object OBJ may be detected by the detector PD. In this case, to prevent light, emitted by the light sources R, G, and B, from being directly incident on the detector PD, a partition wall 213 for blocking light may be disposed between the light sources R, G, and B and the detector PD.

In another example, as illustrated in FIG. 2C, the multi-wavelength light sources R, G, and B and the detectors PD may be arranged in multiple layers. As illustrated in FIG. 2C, the multi-wavelength light sources R, G, and B may be disposed on an upper end or a first surface of the pixel circuit board 212, in which case the multi-wavelength light sources R, G, and B may be patterned on the upper end of the pixel circuit board 212. Further, the detectors PD may be disposed on a lower end or a second surface of the pixel circuit board 212. The detectors PD may be disposed on the lower surface of the pixel circuit board 212 while being in contact with each other or spaced apart from each other. In this case, the pixel circuit board 212 may have a light-transmitting region so that light scattered or reflected from the object may pass through the window 211 to be directed toward the detectors PD.

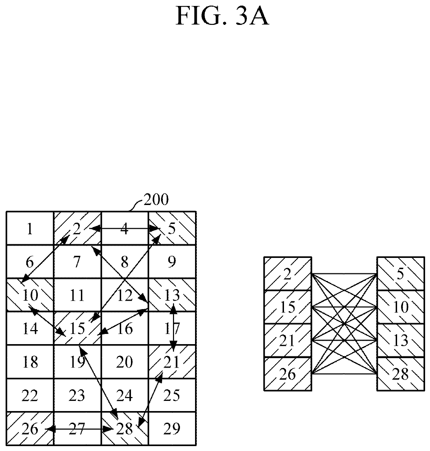

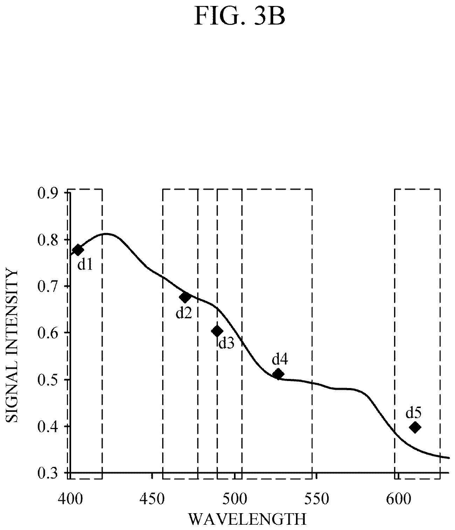

FIGS. 3A and 3B are diagrams explaining examples of driving unit pixels and generating spectra, according to embodiments.

Referring to FIG. 3A, once the object comes into contact with the display for requesting estimation of bio-information, the processor 120 may determine one or more source pixels 2, 15, 21, and 26 and one or more detector pixels 5, 10, 13, and 28 in a unit pixel array 200 for measuring signals from the object. By combining light of determined source pixels 2, 15, 21, and 26 and signals of determined detector pixels 5, 10, 13, and 28 located at various distances, the processor 120 may measure signals at various positions and/or depths of the object, thereby improving accuracy in estimating bio-information.

In addition, the processor 120 may set a partial area for measuring a light signal in the unit pixel array 200, and may measure a light signal in the set partial area. For example, when the object comes into contact with the window of the display 110, the processor 120 may detect a contact position (e.g., fingerprint center point of a finger) of the object, and may set the partial area for determining source pixels and unit pixels based on the detected contact position of the object. In another example, the partial area for measuring the light signal may be preset. In this case, the processor 120 may display an identification mark and the like, indicating the partial area, on the display 110. In this case, a position, size, and the like of the preset area may be changed by a user.

FIG. 3B illustrates a spectrum generated based on signals d1, d2, d3, d4, and d5 of five wavelength ranges that are detected by the detector pixels after emitting light, corresponding to the five wavelength ranges, by color modulation of light sources of source pixels. By combining operation of the determined source pixels and the determined detector pixels, the processor 120 may obtain a plurality of spectra. When obtaining the plurality of spectra as described above, the processor 120 may exclude an invalid spectrum by verifying validity of the spectra and the like. In this case, validity may be verified by calculating a similarity between each of the obtained plurality of spectra and a reference spectrum, and by determining a spectrum having the calculated similarity that exceeds a predetermined threshold, to be a valid spectrum. However, a method of verifying validity is not limited thereto.

In this case, the processor 120 may calculate the similarity by using various similarity calculation algorithms, such as Euclidean distance, Manhattan Distance, Cosine Distance, Mahalanobis Distance, Jaccard Coefficient, Extended Jaccard Coefficient, Pearson's Correlation Coefficient, Spearman's Correlation Coefficient, and the like.

The processor 120 may extract features for estimating bio-information from the obtained spectra, and may estimate bio-information by using an estimation model that defines a correlation between the extracted features and bio-information. In this case, the estimation model may be defined by a linear or non-linear equation, but is not limited thereto.

For example, the processor 120 may extract features by combining an intensity of a signal detected in a reference wavelength range and an intensity of a signal detected in another wavelength range. In this case, the reference wavelength range may be set differently according to a type of bio-information and the like. For example, in the case of estimating carotenoid, the processor 120 may determine a wavelength range of approximately 470 nm to 510 nm in FIG. 3B to be the reference wavelength range, and may extract a combination (e.g., difference or ratio) of the intensity of a signal d3 detected in the reference wavelength range and the intensity of a signal (e.g., d2 and/or d4) detected in an adjacent wavelength range as a feature for estimating carotenoid. However, these are examples.

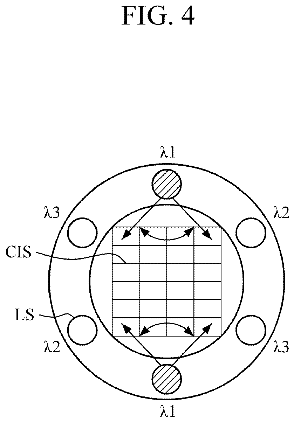

FIG. 4 is a diagram explaining another example of a display, according to an embodiment.

Referring to FIG. 4, the display 110 may include an image sensor CIS and a plurality of light sources LSs.

The light source may include a light emitting diode (LED), a laser diode (LD), a phosphor, and the like. The plurality of light sources may be configured to emit light of different wavelengths, e.g., an infrared wavelength, a red wavelength, a green wavelength, a blue wavelength, a white wavelength, and the like. In this case, each of the plurality of light sources may emit light of different wavelengths λ1 - λ3, or a plurality of light sources may be included for each wavelength and may be disposed at opposite positions as illustrated in FIG. 4. The image sensor may be a complementary metal-oxide semiconductor (CMOS) image sensor. As illustrated in FIG. 4, the plurality of light sources may be disposed around the image sensor. The light sources may be arranged in a linear, circular, elliptical, and polygonal shape, or the like with no particular limitation.

By time-division of all the plurality of light sources, the processor 120 may sequentially drive all the plurality of light sources in a pre-defined direction, such as a clockwise direction, a counter-clockwise direction, a zigzag direction, and the like. Alternatively, the processor 120 may sequentially drive all the plurality of light sources in an order from short to long wavelengths or vice versa. Further, the processor 120 may select some of the plurality of light sources according to a measurement position or a measurement depth of the object and the like, and may sequentially drive the selected light sources in a predetermined direction or in predetermined order of wavelength. By combining the plurality of light sources, the processor 120 may measure light signals at various depths of the object.

The processor 120 may calculate absorbance for each pixel based on pixel data detected by the image sensor after driving the plurality of light sources, e.g., based on the intensity of light received by each pixel of the image sensor, and may estimate bio-information based on the absorbance of each pixel. For example, the processor 120 may estimate bio-information by using an estimation model that defines a correlation between the absorbance and bio-information. In this case, the processor 120 may obtain the absorbance in a proper wavelength range according to a type of bio-information, and may estimate bio-information by using the absorbance in the wavelength range.

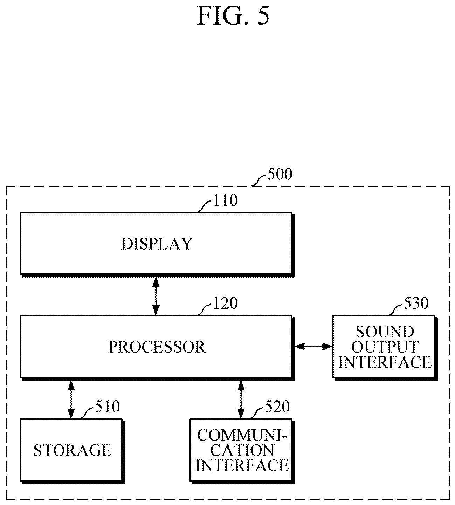

FIG. 5 is a block diagram of an apparatus for estimating bio-information, according to another embodiment.

Referring to FIG. 5, the apparatus 500 for estimating bio-information may include the display 110, the processor 120, a storage 510, a communication interface 520, and a sound output interface 530. The display 110 and the processor 120 are described above in detail, such that the following description will be focused on non-overlapping functions.

The display 110 may output, for example, an interface for supporting various functions of the apparatus 500 for estimating bio-information, and may display data, such as an estimated bio-information value processed by the processor 120 and the like, through the interface. Further, by visually displaying a contact position, in which a user may be required to place an object, and the like, the display 110 may guide the user on the contact position.

In addition, the display 110 may include a touch circuity adapted to detect a touch, and/or sensor circuitry (e.g., pressure sensor, etc.) adapted to measure the intensity of force incurred by the touch. The display 110 may detect a user's touch input by the touch circuit, and may transmit a user's request to the processor 120. Furthermore, when the object comes into contact with the window of the display 110 and applies force thereto, contact pressure may be measured by the pressure sensor and the like of the display 110.

The processor 120 may be electrically connected to the display 110, and may properly control the unit pixels of the display 110 to output image data (e.g., guide information for estimating bio-information, a bio-information estimation result, or other image data) and/or to measure light signals.

For example, the processor 120 may measure light signals or output image data by using the unit pixels of the entire area of the display 110. Alternatively, by dividing the display 110 into a first area for measuring light signals and a second area for outputting the image data, the processor 120 may measure the light signals by using the unit pixels of the first area, and at the same time may output the image data by using the light sources of all the unit pixels of the second area.

The storage 510 may store data related to estimating bio-information. For example, the data may include user characteristic information, such as a user's age, gender, health condition, and the like, color modulation pattern, light source driving conditions and/or an estimation model, and the like. Further, the data may include other data processed or generated by the display 110 and/or the processor 120, application programs or algorithms for estimating bio-information and/or performing other functions, or input data and/or output data about related instructions.

The storage 510 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The communication interface 520 may communicate with an external device by using various wired and wireless communication modules. The communication interface 520 may receive data for estimating bio-information from the external device, and may transmit data processed and/or processed by the display 110 or the processor 120 to the external device. In this case, the external device may include an information processing device such as a smartphone, a tablet PC, a desktop computer, a laptop computer, and the like. In this case, examples of the wired and wireless communication modules may include Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is an example and is not intended to be limiting.

The sound output interface 530 may output sound signals to the outside. The sound output interface 530 may include a speaker and/or a receiver. For example, the processor 120 may convert the estimated bio-information result and/or guide information on the contact position or contact pressure into sound signals, and the sound output interface 530 may output the sound signals.

In addition, the apparatus 500 for estimating bio-information may further include an inputter. The inputter may receive instructions to be used by the processor 120 and the like of the apparatus 500 for estimating bio-information and/or data from a user and the like. The inputter may include a microphone, a mouse, a keyboard, and/or a digital pen (e.g., stylus pen, etc.).

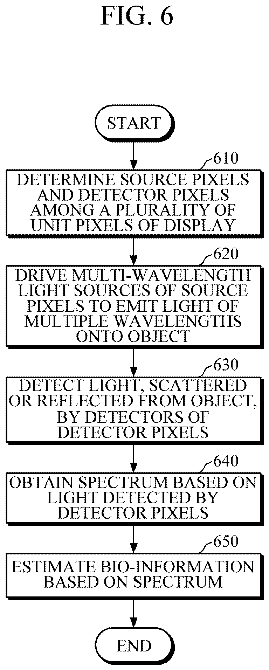

FIG. 6 is a flowchart of a method of estimating bio-information, according to an embodiment.

The method of FIG. 6 is an example of a method of estimating bio-information that is performed by the apparatuses 100 and 500 for estimating bio-information according to the above embodiments, which are described above in detail and thus will be briefly described below to avoid redundancy.

In operation 610, once an object comes into contact with a display, the apparatus for estimating bio-information may determine source pixels and detector pixels among a plurality of unit pixels of the display. In this case, the apparatus for estimating bio-information may determine the source pixels and the detector pixels at different positions. By considering a contact position of the object, a type of bio-information, etc., the apparatus for estimating bio-information may determine the source pixels and the detector pixels by combining the source pixels and the detector pixels at various distances.

In operation 620, the apparatus for estimating bio-information may drive multi-wavelength light sources of the source pixels to emit light of multiple wavelengths onto the object. In operation 630, the apparatus for estimating bio-information may detect light, scattered or reflected from the object, by detectors of the detector pixels. The multi-wavelength light sources may include red, green, and blue light sources. By modulation of various colors, the apparatus for estimating bio-information may sequentially or simultaneously drive the light sources to include proper wavelength ranges for estimating bio-information.

In operation 640, the apparatus for estimating bio-information may obtain a spectrum based on light detected by the detector pixels. In operation 650, the apparatus for estimating bio-information may estimate bio-information based on the obtained spectrum. In this case, when obtaining a plurality of spectra, the apparatus for estimating bio-information may determine a valid spectrum by verifying validity of the spectra. Further, the apparatus for estimating bio-information may extract features from the spectra and may estimate bio-information by applying an estimation model. In this case, the apparatus for estimating bio-information may extract the features by a combination, such as a difference or a ratio between a signal of a first wavelength and a signal of a second wavelength serving as a reference, and the like.

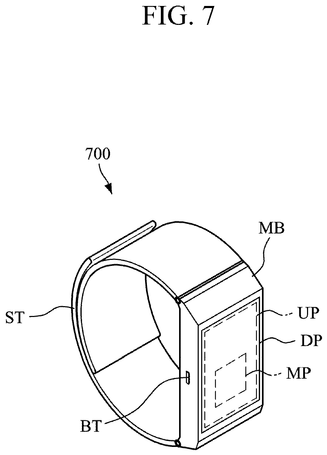

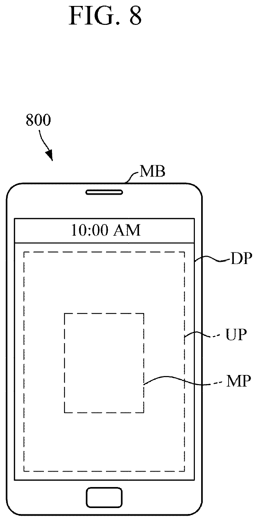

FIGS. 7 and 8 are diagrams illustrating examples of electronic devices having an apparatus for estimating bio-information according to embodiments.

Referring to FIG. 7, the electronic device may be implemented as a wristwatch-type wearable device 700. Here, the wristwatch-type wearable device 700 is an example, and the shape is not particularly limited as long as the wearable device may be worn on the human body. The wristwatch-type wearable device 700 may include a main body MB and a strap ST. The main body MB may have various shapes (e.g., circle, square, etc.), and the strap ST may be made of a flexible material to be wrapped around a user's wrist so that the main body MB may be worn on the wrist. A display DP is disposed on a front surface of the main body MB, and a manipulator BT for receiving a user's instruction may be disposed on a side surface of the main body MB. In this case, the display DP may be an OLED-based display.

Referring to FIG. 8, the electronic device may be implemented as a mobile device 800 such as a smartphone. A display DP may be disposed on the front surface of a main body MB of the mobile device 800, and various modules for processing a user's instructions may be disposed on the front surface, the rear surface, and/or the side surface of the main body MB. In this case, the display DP may be an OLED-based display.

However, the electronic device is not limited to the wearable device 700 or the mobile device 800 illustrated in FIGS. 7 and 8, and examples of the electronic device may include an information processing device, such as a laptop computer having a display, or an Internet of things (IoT) device such as home appliances including a refrigerator, a TV, and the like.

Referring to FIGS. 7 and 8, the main body MB may include a processor, a memory, a sound output device, a sensor module, a haptic module, a camera module, a power management module, a battery, a communication module, and the like. Some of the components may be omitted from the electronic device, and other components may be added.

At least some of the functions of the apparatuses 100 and 500 for estimating bio-information may be implemented as single integrated circuitry to be mounted in the sensor module of the electronic devices 700 and 800, or may be distributed in different components. For example, the functions of the display 110 and the processor 120 of the aforementioned apparatuses 100 and 500 for estimating bio-information may be included in the display MB and the processor of the wearable device 700, respectively.

A plurality of unit pixels capable of measuring light signals from the object may be arranged in the entire area UP of the display DP. However, the display DP is not limited thereto, and unit pixels for measuring the light signals may be arranged in a partial area MP of the display DP. Each unit pixel may include light sources emitting light of red, green, and blue wavelengths, and a detector for detecting light signals.

Once a request for estimating bio-information is received through touch input of the display DP or by the manipulator BP, and when the object comes into contact with the display DP, the processor may determine source pixels and detector pixels among the plurality of unit pixels and may obtain various spectra by driving the source pixels and the detector pixels by combining the source pixels and the detector pixels at various distances. By color modulation of multi-wavelength light sources of the source pixels according to a type of bio-information and the like, the processor may measure light signals in a wavelength range.

In this case, the processor may detect a contact position of the object by sequentially driving all the unit pixels, and may also measure the light signals by using the unit pixels of a partial area (e.g., MP) based on the detected contact position. Alternatively, in order for the object to come into contact with the partial area (e.g., MP) of the display DP, the processor may output guide information in a remaining area of the display DP. In this case, the processor may extract a user's fingerprint information based on the light signals detected in the partial area MP, and may perform user authentication by using the extracted fingerprint information.

Data generated and/or processed by the electronic devices 700 and 800 may be stored in the memory, and the data stored in the memory may be used by the processor or other components of the electronic devices 700 and 800.

The data generated and/or processed by the processor or other components of the electronic devices 700 and 800 may be converted into sound signals, to be output by the sound output device, and may be output by the haptic module as a mechanical stimulus (e.g., vibration, motion, etc.) or an electrical stimulus that may be recognized by a user by tactile sensation or kinesthetic sensation.

The communication module may support communication of the electronic devices 700 and 800 with other devices, e.g., a mobile device, a smartphone, a laptop computer, etc. that are located in a network environment. The data generated and/or processed by the processor or other components of the electronic devices 700 and 800 may be transmitted to other devices through the communication module, and data for estimating bio-information and the like may be received from other devices and stored in the memory.

Example embodiments can be realized as a computer-readable code written on a computer-readable recording medium. The computer-readable recording medium may be any type of recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium can be distributed over a plurality of computer systems connected to a network so that a computer-readable code is written thereto and executed therefrom in a decentralized manner. Functional programs, codes, and code segments for realizing example embodiments can be easily deduced by computer programmers of ordinary skill in the art, to which example embodiments pertain.

The inventive concepts have been described herein with regard to the embodiments. However, it will be obvious to those skilled in the art that various changes and modifications can be made without changing technical ideas and features. Thus, it is clear that the above-described embodiments are illustrative in all aspects and are not intended to limit the inventive concepts.

## Claims

1. An apparatus (100; 500) for estimating bio-information, the apparatus comprising:
a display (110) comprising unit pixels (1, ..., 29), each of the unit pixels comprising:
light sources configured to emit light having different wavelengths; and
one or more detectors (PD) configured to detect light having the different wavelengths; and
a processor (120) configured to:
detect a contact position of an object (OBJ) on the display;
determine a partial area of the display, based on the detected contact position;
determine source pixels configured to emit light onto the object, among the unit pixels, based on the determined partial area;
determine detector pixels configured to detect light that is scattered or reflected from the object, among the unit pixels, based on the determined partial area;
control the determined source pixels and the determined detector pixels to obtain a spectrum based on the light having multiple wavelengths that is detected by the detector pixels; and
estimate the bio-information, based on the obtained spectrum.

2. The apparatus (100; 500) of claim 1, wherein the light sources comprise a red light source, a green light source, and a blue light source.

3. The apparatus (100; 500) of claim 1 or2, wherein the one or more detectors (PD) comprise either one or both of a photodiode and a Complementary Metal Oxide Semiconductor, CMOS, image sensor.

4. The apparatus (100; 500) of one of claims 1 to 3, wherein the light sources and the one or more detectors (PD) are disposed on a same surface of a pixel circuit board (212).

5. The apparatus (100; 500) of claim 4, wherein the light sources and the one or more detectors (PD) are patterned on the same surface of the pixel circuit board (212).

6. The apparatus (100; 500) of claim 4, wherein a partition wall (213) for blocking light is interposed between the light sources and the one or more detectors (PD).

7. The apparatus (100; 500) of one of claims 1 to 6, wherein the light sources are disposed on a first surface of a pixel circuit board (212), and
wherein the one or more detectors (PD) are disposed on a second surface of the pixel circuit board, the second surface being opposite to the first surface.

8. The apparatus (100; 500) of one of claims 1 to 7, wherein the processor (120) is further configured to determine the source pixels and the detector pixels at different positions, among the unit pixels (1, ..., 29).

9. The apparatus (100; 500) of claim 8, wherein the source pixels and the detector pixels are further determined, based on any one or any combination of a type of the bio-information, a light path, and an allowable range of Signal-to-Noise Ratio, SNR, values.

10. The apparatus (100; 500) of one of claims 1 to 9, wherein the processor (120) is further configured to perform color modulation of the light sources of the determined source pixels, based on a type of the bio-information.

11. The apparatus (100; 500) of claim 10, wherein the processor (120) is further configured to perform the color modulation of the light sources of the determined source pixels, to include a wavelength range of 470 nm to 510 nm.

12. The apparatus (100; 500) of one of claims 1 to 11, wherein the processor (120) is further configured to:
extract a light intensity for each of the different wavelengths, based on Full Width Half Maximum, FWHM, characteristics of the light sources emitting light of each wavelength; and
obtain the spectrum, based on the extracted light intensity for each of the different wavelengths.

13. The apparatus (100; 500) of one of claims 1 to 12, wherein the bio-information comprises any one or any combination of skin carotenoid, blood carotenoid, glucose, urea, lactate, triglyceride, total protein, cholesterol, and ethanol.

14. A method of estimating bio-information, the method comprising:
detecting a contact position of an object (OBJ) on a display (110), the display comprising unit pixels (1, ..., 29), each of the unit pixels comprising light sources emitting light having different wavelengths and one or more detectors (PD) detecting light having the different wavelengths;
determining a partial area of the display, based on the contact position;
determining source pixels and detector pixels, among the unit pixels, based on the determined partial area;
controlling the determined source pixels to emit light of onto the object;
controlling the determined detector pixels to detect light that is scattered or reflected from the object;
obtaining a spectrum based on the light having multiple wavelengths that is detected by the detector pixels; and
estimating the bio-information, based on the obtained spectrum.

15. A computer-readable storage medium storing instructions that, when executed by a processor (120) of an apparatus (100; 500) for estimating bio-information that comprises a display (110) comprising unit pixels (1, ..., 29), each of the unit pixels comprising light sources configured to emit light having different wavelengths, at least one detector (PD) configured to detect light having the different wavelengths, and the processor, cause the processor to:
detect a contact position of an object (OBJ) on the display;
determine a partial area of the display, based on the detected contact position;
determine source pixels configured to emit light onto the object, among the unit pixels, based on the determined partial area;
determine detector pixels configured to detect light that is scattered or reflected from the object, among the unit pixels, based on the determined partial area;
control the determined source pixels and the determined detector pixels to obtain a spectrum based on the light having multiple wavelengths that is detected by the detector pixels; and
estimate the bio-information, based on the obtained spectrum.

## Patentansprüche

1. Vorrichtung (100; 500) zum Schätzen von Bioinformationen, wobei die Vorrichtung umfasst:
eine Anzeige (110), umfassend Einheitspixel (1, ..., 29), wobei jedes der Einheitspixel umfasst:
zum Ausstrahlen von Licht mit verschiedenen Wellenlängen ausgebildete Lichtquellen; und
einen oder mehrere zum Erfassen von Licht mit den verschiedenen Wellenlängen ausgebildete Detektoren (PD); und
einen Prozessor (120), ausgebildet zum:
Erfassen einer Kontaktposition eines Objekts (OBJ) auf der Anzeige;
Bestimmen einer Teilfläche der Anzeige auf Basis der erfassten Kontaktposition;
Bestimmen von Quellpixeln, ausgebildet zum Ausstrahlen von Licht auf das Objekt, von den Einheitspixeln auf Basis der bestimmten Teilfläche;
Bestimmen von Detektorpixeln, ausgebildet zum Erfassen von Licht, das vom Objekt gestreut oder reflektiert wird, von den Einheitspixeln auf Basis der bestimmten Teilfläche;
Steuern der bestimmten Quellpixel und der bestimmten Detektorpixel zum Ermitteln eines Spektrums auf Basis des Lichts mit mehreren Wellenlängen, das von den Detektorpixeln erfasst wird; und
Schätzen der Bioinformationen auf Basis des ermittelten Spektrums.

2. Vorrichtung (100; 500) nach Anspruch 1, wobei die Lichtquellen eine rote Lichtquelle, eine grüne Lichtquelle und eine blaue Lichtquelle umfassen.

3. Vorrichtung (100; 500) nach Anspruch 1 oder 2, wobei der eine oder die mehreren Detektoren (PD) eine Fotodiode oder/und einen Complementary-Metal-Oxide-Semiconductor-(CMOS-)Bildsensor umfassen.

4. Vorrichtung (100; 500) nach einem der Ansprüche 1 bis 3, wobei die Lichtquellen und der eine oder die mehreren Detektoren (PD) auf einer gleichen Fläche einer Pixelleiterplatte (212) angeordnet sind.

5. Vorrichtung (100; 500) nach Anspruch 4, wobei die Lichtquellen und der eine oder die mehreren Detektoren (PD) auf der gleichen Fläche der Pixelleiterplatte (212) gemustert sind.

6. Vorrichtung (100; 500) nach Anspruch 4, wobei eine Trennwand (213) zum Blockieren von Licht zwischen den Lichtquellen und dem einen oder den mehreren Detektoren (PD) angeordnet ist.

7. Vorrichtung (100; 500) nach einem der Ansprüche 1 bis 6, wobei die Lichtquellen auf einer ersten Fläche einer Pixelleiterplatte (212) angeordnet sind, und
wobei der eine oder die mehreren Detektoren (PD) auf einer zweiten Fläche der Pixelleiterplatte angeordnet sind, wobei die zweite Fläche der ersten Fläche gegenüberliegt.

8. Vorrichtung (100; 500) nach einem der Ansprüche 1 bis 7, wobei der Prozessor (120) ferner zum Bestimmen der Quellpixel und der Detektorpixel an verschiedenen Positionen von den Einheitspixeln (1, ..., 29) ausgebildet ist.

9. Vorrichtung (100; 500) nach Anspruch 8, wobei die Quellpixel und die Detektorpixel ferner auf Basis von einem Typ der Bioinformationen, einem Lichtweg oder einem zulässigen Bereich von Signal-to-Noise Ratio-(SNR-)Werten oder einer Kombination hiervon bestimmt werden.

10. Vorrichtung (100; 500) nach einem der Ansprüche 1 bis 9, wobei der Prozessor (120) ferner zum Durchführen einer Farbmodulation der Lichtquellen der bestimmten Quellpixel auf Basis eines Typs der Bioinformationen ausgebildet ist.

11. Vorrichtung (100; 500) nach Anspruch 10, wobei der Prozessor (120) ferner zum Durchführen der Farbmodulation der Lichtquellen der bestimmten Quellpixel, so dass ein Wellenlängenbereich von 470 nm bis 510 nm enthalten ist, ausgebildet ist.

12. Vorrichtung (100; 500) nach einem der Ansprüche 1 bis 11, wobei der Prozessor (120) ferner ausgebildet ist zum:
Extrahieren einer Lichtstärke für jede der verschiedenen Wellenlängen auf Basis von Full-Width-Half-Maximum-(FWHM-)Merkmalen der Licht von jeder Wellenlänge ausstrahlenden Lichtquellen; und
Ermitteln des Spektrums auf Basis der extrahierten Lichtstärke für jede der verschiedenen Wellenlängen.

13. Vorrichtung (100; 500) nach einem der Ansprüche 1 bis 12, wobei die Bioinformationen Haut-Carotinoid, Blut-Carotinoid, Glukose, Harnstoff, Laktat, Triglycerid, Gesamtprotein, Cholesterin und Ethanol umfassen.

14. Verfahren zum Schätzen von Bioinformationen, wobei das Verfahren umfasst:
Erfassen einer Kontaktposition eines Objekts (OBJ) auf einer Anzeige (110), wobei die Anzeige Einheitspixel (1, ..., 29) umfasst, wobei jedes der Einheitspixel Licht mit verschiedenen Wellenlängen ausstrahlende Lichtquellen und einen oder mehrere Licht mit den verschiedenen Wellenlängen erfassende Detektoren (PD) umfasst;
Bestimmen einer Teilfläche der Anzeige auf Basis der Kontaktposition;
Bestimmen von Quellpixeln und Detektorpixeln von den Einheitspixeln auf Basis der bestimmten Teilfläche;
Steuern der bestimmten Quellpixel zum Ausstrahlen von Licht auf das Objekt;
Steuern der bestimmten Detektorpixel zum Erfassen von Licht, das vom Objekt gestreut oder reflektiert wird;
Ermitteln eines Spektrums auf Basis des Lichts mit mehreren Wellenlängen, das von den Detektorpixeln erfasst wird; und
Schätzen der Bioinformationen auf Basis des ermittelten Spektrums.

15. Computerlesbares Speichermedium zum Speichern von Anweisungen, die, wenn von einem Prozessor (120) einer Vorrichtung (100; 500) zum Schätzen von Bioinformationen ausgeführt, die eine Anzeige (110), umfassend Einheitspixel (1, 29) umfasst, wobei jedes der Einheitspixel Lichtquellen, ausgebildet zum Ausstrahlen von Licht mit verschiedenen Wellenlängen, wenigstens einen Detektor (PD), ausgebildet zum Erfassen von Licht mit den verschiedenen Wellenlängen, und den Prozessor umfasst, den Prozessor veranlassen zum:
Erfassen einer Kontaktposition eines Objekts (OBJ) auf der Anzeige;
Bestimmen einer Teilfläche der Anzeige auf Basis der erfassten Kontaktposition;
Bestimmen von Quellpixeln, ausgebildet zum Ausstrahlen von Licht auf das Objekt, von den Einheitspixeln auf Basis der bestimmten Teilfläche;
Bestimmen von Detektorpixeln, ausgebildet zum Erfassen von Licht, das vom Objekt gestreut oder reflektiert wird, von den Einheitspixeln auf Basis der bestimmten Teilfläche;
Steuern der bestimmten Quellpixel und der bestimmten Detektorpixel zum Ermitteln eines Spektrums auf Basis des Lichts mit mehreren Wellenlängen, das von den Detektorpixeln erfasst wird; und
Schätzen der Bioinformationen auf Basis des ermittelten Spektrums.

## Revendications

1. Appareil (100 ; 500) permettant d'estimer les informations biologiques, l'appareil comprenant :
un écran (110) comprenant des pixels unitaires (1, ..., 29), chacun des pixels unitaires comprenant :
des sources de lumière configurées pour émettre de la lumière ayant différentes longueurs d'onde ; et
un ou plusieurs détecteurs (PD) configurés pour détecter la lumière ayant les différentes longueurs d'onde ; et
un processeur (120) configuré pour :
détecter la position de contact d'un objet (OBJ) sur l'écran ;
déterminer une zone partielle de l'écran, sur la base de la position de contact détectée ;
déterminer les pixels sources configurés pour émettre de la lumière sur l'objet, parmi les pixels unitaires, en fonction de la zone partielle déterminée ;
déterminer les pixels de détection configurés pour détecter la lumière diffusée ou réfléchie par l'objet, parmi les pixels de l'unité, sur la base de la zone partielle déterminée ;
commander les pixels sources déterminés et les pixels détecteurs déterminés pour obtenir un spectre basé sur la lumière ayant plusieurs longueurs d'onde qui est détectée par les pixels détecteurs ; et
estimer les informations biologiques sur la base du spectre obtenu.

2. Appareil (100 ; 500) selon la revendication 1, dans lequel les sources de lumière comprennent une source de lumière rouge, une source de lumière verte et une source de lumière bleue.

3. Appareil (100 ; 500) selon la revendication 1 ou 2, dans lequel les un ou plusieurs détecteurs (PD) comprennent une photodiode ou un capteur d'image à semiconducteur complémentaire à oxyde métallique, CMOS, ou les deux.

4. Appareil (100 ; 500) selon l'une quelconque des revendications 1 à 3, dans lequel les sources de lumière et les un ou plusieurs détecteurs (PD) sont disposés sur une même surface d'une carte de circuit imprimé de pixels (212).

5. Appareil (100 ; 500) selon la revendication 4, dans lequel les sources de lumière et les un ou plusieurs détecteurs (PD) sont disposés sur la même surface de la carte de circuit imprimé de pixels (212).

6. Appareil (100 ; 500) selon la revendication 4, dans lequel une cloison (213) pour bloquer la lumière est interposée entre les sources de lumière et les un ou plusieurs détecteurs (PD).

7. Appareil (100 ; 500) selon l'une quelconque des revendications 1 à 6, dans lequel les sources de lumière sont disposées sur une première surface d'une carte de circuit imprimé de pixels (212), et
dans lequel un ou plusieurs détecteurs (PD) sont disposés sur une deuxième surface de la carte de circuit imprimé de pixels, la deuxième surface étant opposée à la première.

8. Appareil (100 ; 500) selon l'une quelconque des revendications 1 à 7, dans lequel le processeur (120) est en outre configuré pour déterminer les pixels sources et les pixels détecteurs à différentes positions, parmi les pixels unitaires (1, ..., 29).

9. Appareil (100 ; 500) selon la revendication 8, dans lequel les pixels sources et les pixels détecteurs sont en outre déterminés sur la base de l'un des éléments suivants ou d'une combinaison de ceux-ci : type d'informations biologiques, trajet de la lumière et plage autorisée de valeurs du rapport signal/bruit (SNR).

10. Appareil (100 ; 500) selon l'une quelconque des revendications 1 à 9, dans lequel le processeur (120) est en outre configuré pour effectuer une modulation de couleur des sources de lumière des pixels sources déterminés, sur la base d'un type d'informations biologiques.

11. Appareil (100 ; 500) selon la revendication 10, dans lequel le processeur (120) est en outre configuré pour effectuer la modulation de couleur des sources de lumière des pixels sources déterminés, pour inclure une plage de longueurs d'onde de 470 nm à 510 nm.

12. Appareil (100 ; 500) selon l'une quelconque des revendications 1 à 11, dans lequel le processeur (120) est en outre configuré pour :
extraire une intensité lumineuse pour chacune des différentes longueurs d'onde, sur la base des caractéristiques Full Width Half Maximum (FWHM) des sources de lumière émettant de la lumière de chaque longueur d'onde ; et
obtenir le spectre, sur la base de l'intensité lumineuse extraite pour chacune des différentes longueurs d'onde.

13. Appareil (100 ; 500) selon l'une quelconque des revendications 1 à 12, dans lequel les informations biologiques comprennent une ou plusieurs combinaisons de caroténoïdes de la peau, de caroténoïdes du sang, de glucose, d'urée, de lactate, de triglycérides, de protéines totales, de cholestérol et d'éthanol.

14. Procédé d'estimation des informations biologiques, le procédé comprenant les étapes consistant à :
détecter une position de contact d'un objet (OBJ) sur un écran (110), l'écran comprenant des pixels unitaires (1, ..., 29), chacun des pixels unitaires comprenant des sources de lumière émettant une lumière ayant différentes longueurs d'onde et un ou plusieurs détecteurs (PD) détectant la lumière ayant les différentes longueurs d'onde ;
déterminer une zone partielle de l'écran, en fonction de la position de contact ;
déterminer les pixels sources et les pixels détecteurs, parmi les pixels unitaires, sur la base de la zone partielle déterminée ;
commander les pixels source déterminée pour qu'ils émettent de la lumière sur l'objet ;
commander les pixels détecteurs déterminés pour détecter la lumière diffusée ou réfléchie par l'objet ;
obtenir un spectre basé sur la lumière ayant plusieurs longueurs d'onde qui est détectée par les pixels détecteurs ; et
estimer des informations biologiques, sur la base du spectre obtenu.

15. Support de stockage lisible par ordinateur stockant des instructions qui, lorsqu'elles sont exécutées par un processeur (120) d'un appareil (100 ; 500) pour estimer des informations biologiques qui comprennent un écran (110) comprenant des pixels unitaires (1, ..., 29), chacun des pixels unitaires comprenant des sources de lumière configurées pour émettre de la lumière ayant différentes longueurs d'onde, au moins un détecteur (PD) configuré pour détecter la lumière ayant les différentes longueurs d'onde, et le processeur, amènent le processeur à :
détecter la position de contact d'un objet (OBJ) sur l'écran ;
déterminer une zone partielle de l'écran, sur la base de la position de contact détectée ;
déterminer les pixels sources configurés pour émettre de la lumière sur l'objet, parmi les pixels unitaires, en fonction de la zone partielle déterminée ;
déterminer les pixels de détection configurés pour détecter la lumière diffusée ou réfléchie par l'objet, parmi les pixels de l'unité, sur la base de la zone partielle déterminée ;
commander les pixels sources déterminés et les pixels détecteurs déterminés pour obtenir un spectre basé sur la lumière ayant plusieurs longueurs d'onde qui est détectée par les pixels détecteurs ; et
estimer les informations biologiques sur la base du spectre obtenu.
